# EUROPEAN PATENT APPLICATION

(11) **EP 4 000 756 A1**
(43) Date of publication of application: **25.05.2022**
(21) Application number: 21208184.8
(22) Date of filing: 15.11.2021
(51) Int. Cl.: B09C 1/10, C12M 1/00

(54) **DEVICE FOR THE ENVIRONMENTAL RECLAMATION OF CONTAMINATED SOILS**

(30) Priority: 18.11.2020 IT 202000027639
(71) Applicant: Itelyum Regeneration S.p.A., 26854 Pieve Fissiraga (LO) (IT); Sapienza Universita di Roma, 00185 Rome (IT)
(72) Inventor: BONI, Maria Rosaria, 00185 Rome (IT); CECCARELLI, Stefano, 26854 Pieve Fissiraga (IT); DI MARCANTONIO, Camilla, 00185 Rome (IT); GALLO, Francesco, 26854 Pieve Fissiraga (IT); JIRILLO, Jacopo, 26854 Pieve Fissiraga (IT); STRACCAMORE, Elisabetta, 26854 Pieve Fissiraga (IT); CHIAVOLA, Agostina, 00185 Roma (IT); MARZEDDU, Simone, 00185 Rome (IT)
(74) Representative: Vatti, Francesco Paolo

(57) **Abstract**

A device is described for the environmental reclamation of contaminated soils through the technology known as landfarming. According to the invention, such device provides one or more tubs (1) of a rigid material, (for example a metal material or a plastic material) each equipped with a sensing probe (3) of soil moisture to be inserted thereinto for reclamation, a watering unit (5), connected to said probe (3), and a collection point (4) of soil sample to allow analysis thereof.

Preferably, the tub (1) has a bottom plane (2) tilted of 0.01% to 5%.

## Description

The present invention relates to a stationary device for environmental reclamation of contaminated soils, particularly intended for the process known as "landfarming".

Since a certain ecological awareness arose in people due to the development of industries and to the pollution that was its negative by-product, one of the most urgent problems is the removal of pollutants. This happens at all levels: air, water, and soil. Soil contamination, although being the least mentioned, can cause several negative effects. In particular, it can lead to contamination of underlying drinking water, or to making the agricultural crops (in terms of food) produced thereon dangerous for health. Soil contamination may also lead to a release over time of gaseous pollutants into the surrounding atmosphere.

The reclamation of contaminated soils can be performed according to various approaches.

A first subdivision can be the one reported in Italian Legislative Decree no. 152/06: emergency safety measures, operational safety measures, and permanent safety measures.

When looking at permanent safety measures, from interventions in situ, interventions ex situ in the area, and interventions ex situ outside the area can be found.

Among the interventions in situ, the so-called landfarming procedure is particularly relevant. Such procedure provides for the removal of soil pollutants through the action of bacteria and other microorganisms, which degrade them and make them harmless, thereby removing them completely. Basically, the microorganisms feed on the pollutants, so that their removal is largely sustained over time, up to an almost complete degradation.

The soil to be recovered is taken from its seat, brought to a proper area, and laid on a draining bed which is, in turn, placed on a waterproof covering - for example a plastic sheet - which prevents the pollutants from migrating from the reclamation-site and contaminating other soils. The thus deposited soil is then watered with water enriched with oxygen and other nutrients, in order to facilitate the microorganism proliferation and activity. Leachate is thus produced, which is usually recycled to give the microorganisms enough time to complete their work; the leachate can be separately treated before recycling, or it can be recycled directly as it is.

Landfarming comes into two types, depending on how you operate: biostimulation and bioaugmentation. In biostimulation, the used bacterial population is the native one and the process takes place simply by introducing suitable nutrients into the reaction environment, so as to stimulate the growth and development of said bacterial population beyond what it would naturally do. In bioaugmentation, on the contrary, new bacterial species, which are not originally present in the soil to be reclaimed are introduced, selecting the ones most suitable for the purpose to be achieved. Obviously, also in this case nutrients need to be continuously supplied until complete reclamation, for a greater growth of the inoculated bacterial species. Naturally, bioaugmentation leads to a heavy modification, both qualitative and quantitative, of the bacterial population in the soil to be remediated.

In the scope of bioaugmentation, the present Applicant recently filed the Italian patent application no. 102020000020806 which provides the use of sludge coming from the purification of hydrocarbon-contaminated waters as inoculum in the soil to be remediated.

Generally, landfarming is carried out in special tubs dug into the ground by laying a water-resistant sheet and a drainage bed, whereon the soil to be remediated is laid. Generally, such tubs are located quite far from where the sludge to be used as inoculum is taken, both because rather large spaces are required, which are rarely found in industrial sites where spaces are fully exploited, and because at the end of the operations there may be a recontamination of the soil, because of the presence of the substances eliminated by the sludge. This issue becomes particularly serious in situations such as landfarming pilot plants, for which considerable excavation and transport costs are required, with small, if not negligible, amounts of soil to be remediated; in this case, the evaluation of the remediation process progress can also be a criticism, as it is difficult to collect soil samples during the process.

It should also be considered that said tubs are usually dug in large plots of land and - as already mentioned - they are made waterproof, thus creating soil consumption issues, which add up to the already existing problems related to the strong urbanisation which characterises our age.

CN 111 530 905 describes an integrated device for on-site soil reclamation, which includes a moving vehicle, on which a telescopic rod at the front end and a vertical blade at the bottom end are mounted. A rotating platform is arranged on the moving vehicle. An excavation arm is provided on an upright column, with a bucket. A treatment box is arranged on the moving vehicle, whereon a partition plate is placed. On the side wall of the treatment box a water tank is provided with a water pipe at the outlet of which a spraying disk is mounted; a sterilisation lamp is on the inner wall of the treatment box. A pipe for adding bacterial strain is at the upper part of the treatment box there. An air-injection tank is also mounted on the moving vehicle. A rotating drum provided with many turning plates completes it all. The vehicle allows to perform the treatments on-site, without digging and moving soil. Therefore, it is not a stationary device, so that it can be used only for very small amounts of soil, under very favourable orographic conditions, thus being suitable only for domestic environments or small community ones, and not for industrial and extensive contexts. Besides, it does not allow for collecting soil samples during the reclamation, making it impossible to monitor its trend over time. Furthermore, there is no way to adapt to contingent situations the amount of water or microorganisms to be added, as there is no type of sensor.

CN 110 064 646 discloses an integrated, moving system for soil reclamation, which includes a box-like main body carrying a platform at the bottom. On the platform, water collection and suction/ventilation tanks are symmetrically arranged. Perforated pipelines are arranged in the suction/ventilation tanks, covered with water-proof protective layers. Heating means are placed close to the platform. An I-shaped steel beam is placed on a support and groups of sensors and spraying devices, moving on rails, are placed under said beam. Several horizontal-blade stirrers on the two sides of the beam support are arranged inside the box-like main body. An air vent system is provided at the top of the box-like main body and exhaust gases are discharged from the box-like main body to an exhaust gases treatment unit, for purification. Similarly, a water discharge, placed inside the same box-like body, discharges polluted water to a wastewater treatment unit. Supply doors and discharge doors, respectively open upwards and downwards, are arranged on the two sides of the box-like body. Also in this case, the device cannot be used for significant amounts of soil and there is no possibility of collecting soil to monitor the reclamation progress.

KR 101 165 675 refers to an apparatus for spraying a microbial agent to improve the convenience and effectiveness of the oil-decomposition in a contaminated soil, reducing its reclamation time. Such disclosed apparatus includes a bath under stirring, a stirring rod, a propeller, an air-injection unit comprising an air-injection pipe and a spray unit. A screen to adjust the size of solid microbial agents is arranged where the material to be treated enters the bath under stirring. A vibration-inducing device is mounted on the screen to easily introduce the solid microbial agents into the bath under stirring. A plurality of baffle plates is arranged on the side surface of the inner side of the bath under stirring. The air-injection pipe includes a first and a second air-injection pipe, the second being connected to an inlet pipe and to a spray pipe. This is not a device suitable for operations of remediation according to the technology known as landfarming.

CN 106 234 162 refers to an intelligent watering system which includes a plurality of grassy ditches arranged on the green field, a moisture sensor, a main collection barrel and an auxiliary collection barrel, arranged at the lowest end of the green field. The moisture sensor is connected to a controller which regulates and operates the irrigation. This is not a reclamation device.

The problem underlying the present invention is to propose a reactor structure for landfarming operations which overcomes the aforementioned drawbacks and allows to operate in the same place where sludge is produced or, at least, in close proximity, while reducing soil consumption. This purpose is achieved by a stationary device for environmental reclamation of contaminated soils through the technology known as landfarming, characterised in that it provides one or more tubs containing the soil to be reclaimed, into which said soil to be reclaimed is introduced, taking it from its natural seat, and laid flat and levelled, in a similar way to how it is found in its natural location, said tubs being of a rigid material, to be placed resting on the ground or partially buried in it, and being equipped with a sensing probe of soil moisture to be inserted thereinto for reclamation, a watering unit connected to said probe, and a collection point of soil samples for analysis. The dependent claims describe preferred features of the invention.

Further features and advantages of the invention will anyway be better apparent from the following detailed description of a preferred embodiment, given purely by way of non-limiting example and illustrated in the attached drawings, wherein:
Fig. 1 is a schematic, perspective view of a device according to a preferred embodiment of the present invention;
Fig. 2 shows a system which employs devices according to the present invention, based on the embodiment of Fig. 1;
Fig. 3 shows an operating diagram of the system according to Fig. 2; and
Fig. 4 is a detail of Fig. 3.

According to the present invention, the device includes a tub 1 to be laid on a land, placing it above the ground or partially in-ground. The tub 1 is made of rigid material and can preferably be made of a metal material, such as cast iron, iron, steel, aluminium, lead or their alloys, or in a plastic material which is resistant under the treatment conditions, such as polyethylene, polypropylene, polystyrene, polycarbonate, Kevlar, polyethylene terephthalate, polyurethane, polyamide, and others. It can also be made of wood or a stone material as long as the chemical conditions allow it. It is preferred that the tub 1 in made of AISI 316L steel, given its high resistance to corrosion. Preferably, the tub 1 is in the shape of a parallelepiped which allows to place it without causing any space issues.

The tub 1 preferably has a tilted bottom plane 2 for a better irrigation of the soil contained therein. Preferably, the slope of the bottom plane 2 ranges between 0.01% and 5%, preferably ranging from 0.8% to 2% and preferably being around 1%. This range of slope of the soil is to reproduce the slope of the land normally undergoing reclamation, in order to work under similar conditions to the natural ones; in this way, the device according to the present invention could be used for a study of reclamation directly in the land, profiling the reclamation rate depending on soil depths. A tub 1 suitable for use in a pilot plant is, for example, 10 cm x 30 cm x 20 cm in size.

A sensing probe 3 is further placed inside the tub 1. The sensing probe 3 generally and mainly detects the moisture of the soil under reclamation, but sensing probes can be provided that also monitor one or more of the following parameters: temperature, pressure, concentration of microorganisms, concentration of oxygen, concentration of carbon dioxide, so as to better monitor the remediation progress and to quickly and reliably identify the possible causes of a slowing down in the remediation process going on in tub 1, and to be able of acting to remove them. The sensing probe 3 can be of any known type. For example, it can be the EE210 sensing probe, marketed by EE Elektronik of En-gerwitzdorf, Austria.

Externally to the bottom part of the tub 1 a collection point 4 is provided, which can be of any known type, for example a pipe with an opening/closing valve or a tap.

Finally, a watering unit 5 is associated to the tub 1, preferably including a tank 6, a pipeline 7 and a nozzle system 8. The nozzles 8 can preferably consist of pipes 9 with openings 10 along their length. This particular embodiment ensures an even irrigation within the entire tub 1.

The sensing probe 3 and the watering unit 5 are logically connected by a processing unit 11, for example a PLC.

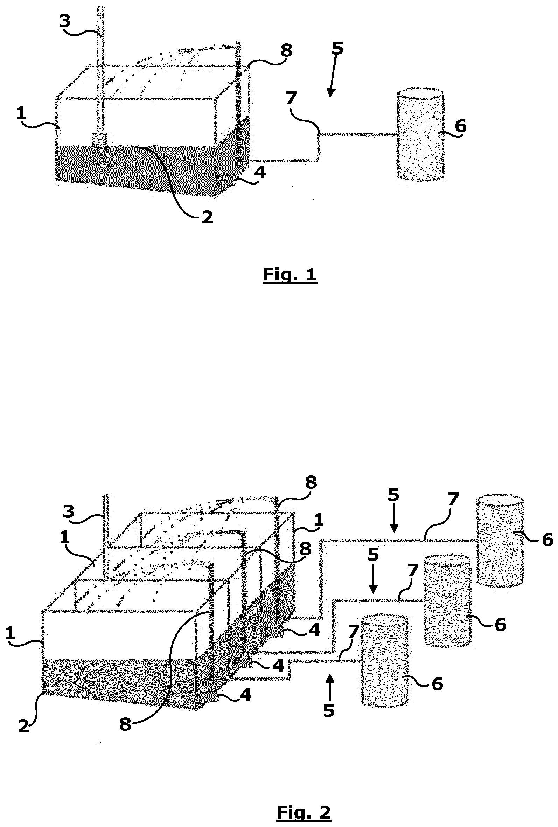

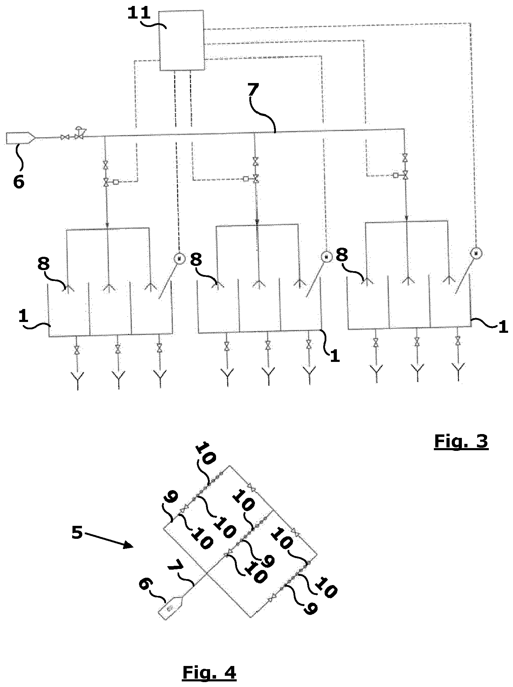

The device according to the present invention can function as a single tub 1, as shown in Fig. 1, or as a system of multiple tubs, as shown in Figs. 2 and 3 (where three tubs are illustrated) . Therefore, the invention also relates to a system for environmental reclamation of contaminated soils including one or more of the devices described so far.

At the start of the process, the soil to be remediated is introduced into the tub(s) 1, making a relatively thin layer (for example, with a thickness ranging between 10 to 50 cm), so as to keep high the mass transfer with the environment, especially of oxygen. The soil is laid flat and levelled, like in the place from which it is taken. The sensing probe 3 is then inserted into the soil and the sludge to be added are inoculated to improve the efficiency of the reclamation process. Thereby, the reclamation process starts, and bacteria begin their work of pollutant degradation in a per se known manner.

As already mentioned, the sensing probe 3 can detect several parameters; in particular, the sensing probe 3 detects the soil moisture and preferably continuously transmits the relative value to the processing unit 11. As soon as the moisture value drops below a certain threshold, the signal sent to the processing unit 11 causes the watering units 5 to be sent a start signal.

It should be noted that, in case of a system like the one shown in Figs. 2 and 3, each sensing probe 3 and each watering unit 5 can also work independently from those of the other tubs, in contrast to what is illustrated in Fig. 2.

Upon starting the watering unit 5, water is drawn from the relative tank 6 and sent through the pipeline 7 to the nozzles 8, from which it comes out and wets the soil laid on the bottom plane 2 of the relative tub 1, irrigating it. This irrigation raises the moisture value of the soil, so that the optimal living conditions for the bacteria active in the reclamation are maintained, in order to keep the speed of the reclamation process high. The sensing probe 3 continues to sense the moisture and to transmit its value to the processing unit 11. When the moisture value exceeds a certain threshold value - normally higher than that previously mentioned - the processing unit 11 transmits a new signal to the watering unit 5, causing it to stop watering the soil through the nozzles 8.

The reclamation process proceeds and the watering unit 5 will be started again at the moment of a new downward crossing of the threshold value, and it will be stopped at the next upward crossing of the other threshold value.

In addition to water, the watering unit 5 can preferably deliver to the soil inside the tubs 1 also the nutrients possibly needed for carrying out the reclamation: this addition plays also a role in the creation of an ideal environment to promote and facilitate bacteria proliferation.

Bacteria degrade the pollutants in the soil, gradually decontaminating it. Any checks on the progress of the reclamation process can be carried out by collecting soil samples from the collection point 4. The collection point can be a pipe with a suitable valve, a septum, a door, a tap or other. Collecting samples through collection point 4 is much easier than in normal landfarming tubs and, owed to its position with respect to the soil to be remediated, the characteristics of the collected sample are those of the soil bulk and not the usually anomalous ones of the surface layer, so that a much more representative sample of the actual situation can be obtained.

As the analysis show that the remediation has successfully completed, sensing probes 3 and watering units 5 are stopped and the remediated soil is recovered to be put back to its original seat. As already mentioned in the introduction of the present disclosure, the device according to the present invention can be used directly on the site from where the sewage sludge to be used as inoculum is taken, so that there is not the always difficult and expensive handling of said sludge - which is in all respects, especially legal, waste - outside the site, and the bacterial loads are exploited to the maximum. Another advantage is that it is possible to take the soil from the surroundings, without need of digging landfarming tubs, thus considerably limiting the consumption of soil, a very sensitive problem nowadays.

The device according to the present invention is particularly useful in the case of landfarming pilot plants, since it allows to operate in situ, with no major transport of material from one site to another one, and it allows to readily monitor the various steps of reclamation, so as to enable the optimisation of its parameters, in order to improve the future operation performances. Therefore, the invention also provides a device which is a pilot plant. In this case, it will be possible to operate under conditions which are close to reality, i.e., using microcosms. If a system like that illustrated in Figs. 2 and 3 is used for the pilot tests, each tub 1 can be managed under different conditions, so as to identify the optimal ones. The device according to the present invention can be likened in many respects to production plants of agricultural composting amendments, since the sludge after reclamation has precisely the function of agricultural amendments, notoriously having fertilising properties.

However, it is understood that the invention should not be considered as limited to the particular arrangement illustrated above, which is only an exemplary embodiment thereof, but that several variants are possible, all within the reach of a person skilled in the art, without thereby departing from the scope of the invention itself, as defined by the following claims. In particular, the device according to the present invention could be configured to be remote controlled.

### REFERENCE LISTING

- 1: Tub
- 2: Bottom plane (of 1)
- 3: Sensing probe
- 4: Collection point
- 5: Watering unit
- 6: Tank (of 5)
- 7: Pipeline (of 5)
- 8: Nozzles (of 9)
- 9: Pipes (of 5)
- 10: Openings (of 9)
- 11: Processing unit

## Claims

1. Stationary device for environmental reclamation of contaminated soils, through the technology known as landfarming, **characterised in that** it provides one or more tubs (1) containing the soil to be reclaimed and into which said soil to be reclaimed is introduced, taking it from its natural seat, and laid flat and levelled, in a similar way to how it is found in its natural location, said tubs being of a rigid material, to be placed resting on the ground or partially buried in it, and equipped with a sensing probe (3) of soil moisture to be inserted thereinto for reclamation, a watering unit (5), connected to said probe (3) and a collection point (4) of soil samples to allow analysis thereof.

2. Device according to claim 1, **characterised in that** the soil to be reclaimed is introduced into the tub(s) (1) making a layer of thickness between 10 and 50 cm.

3. Device according to claim 1 or 2, **characterised in that** said tub (1) is made of a metal material, such as cast iron, iron, steel, aluminium, lead or alloys of the same.

4. Device according to claim 1 or 2, **characterised in that** said tub (1) is made of a plastic material which is resistant under the treatment conditions, such as polyethylene, polypropylene, polystyrene, polycarbonate, Kevlar, polyethylene terephthalate, polyurethane, polyamide.

5. Device according to any of the previous claims, **characterised in that** the tub (1) has a tilted bottom plane (2).

6. Device according to claim 5, **characterised in that** the slope of the bottom plane (2) ranges from 0.01% to 5%, preferably ranging from 0.8% to 2% and preferably being around 1%.

7. Device according to any of the previous claims, **characterised in that** said sensing probe (3) also monitors also one or more of the following parameters: temperature, pressure, concentration of microorganisms, concentration of oxygen, concentration of carbon dioxide.

8. Device according to any of the previous claims, **characterised in that** said watering unit (5) includes a tank (6), a pipeline (7) and a nozzle system (8).

9. Device according to claim 8, **characterised in that** said nozzles (8) consist of pipes (9) with openings (10) along their length.

10. Device according to any of the preceding claims, **characterised in that** it is a pilot plant.

11. System for environmental reclamation of contaminated soils, including one or more devices according to any of the previous claims.
